# EUROPEAN PATENT APPLICATION

(11) **EP 3 616 752 A1**
(43) Date of publication of application: **04.03.2020**
(21) Application number: 18191045.6
(22) Date of filing: 28.08.2018
(51) Int. Cl.: A61N 5/10

(54) **INTENSITY MODULATED PROTON THERAPY PLAN OPTIMIZATION FOR LOCALIZED PLAN DEFICIENCIES**

(71) Applicant: Koninklijke Philips N.V., 5656 AE Eindhoven (NL)
(72) Inventor: RANGANATHAN, Vaitheeswaran, 5656 AE Eindhoven (NL); PERUMAL, Bojarajan, 5656 AE Eindhoven (NL)
(74) Representative: de Haan, Poul Erik

(57) **Abstract**

A method and system for optimizing an IMPT plan are provided, as well as a computer program product for performing, and an arrangement for planning IMPT. For optimizing the IMPT plan, the following steps are performed. An initial IMPT plan is received, along with anatomical image data of the subject to be treated. Critical proton spots are identified in the initial plan using the anatomical image data. At least one local plan deficiency area associated with the identified critical proton spots is generated. A local treatment plan is then generated for the LPDA by applying robust optimization to it. The optimized treatment plan for delivery to the subject to is then generated by combining the local treatment plan with the initial IMPT plan.

## Description

### FIELD OF THE INVENTION

The invention generally relates to planning intentsity modulated proton therapy (IMPT). In particular the invention relates to optimizing an IMPT plan. More specifically, the invention relates to optimizing an IMPT plan with localized plan deficiencies.

### BACKGROUND OF THE INVENTION

In intensity modulated proton therapy (IMPT), target structures in a patient's body, such as tumors, are treated by subjecting them to irradiation by a beam of protons. The treatment is delivered in such a way that the dose that is delivered to the target structures (TSs) is as high as possible, while at the same time the dose delivered to the surrounding healthy tissue and structures, usually referred to as organs at risk (OARs), is as low as possible. A tradeoff between the two will have to be made. For this, clinical goals are provided. Clinical goals in planning the IMPT treatment will specify the requirements for dose to be deliverd to a TS. The clinical goals may also include maximum value for the dose to be delivered to each OAR. Numerical optimization is typically used to determine the best total dose distribution to deliver. From this dose distribution the parameters for delivery are calculated that form the therapy plan.

IMPT plans define a pattern of locations where the protons beams are to be delivered, together with the intensity of the beam for each location. Because of the shape of the proton beam, a high dose is delivered to a relatively small volume. The location where the dose is delivered is referred to as a spot or proton spot.

Due to the shape of the Bragg peak of the proton beam, IMPT has the advantage that the treatment can be delivered with high accuracy. However, due to the characteristic beam shape used in IMPT, IMPT plans are particularly sensitive to errors such as range and setup uncertainties and organ deformations and movement. Failing to account for such errors in the IMPT plan may result in a delivered dose distribution that is inferior to the planned one.

Setup uncertainty is due to a positional shift of the patient, which may cause misalignment of dose contributions from different beam directions. Even with modern immobilization techniques, deviations of several millimeters are still possible. Range uncertainty corresponds to the uncertainty of the location of the Bragg peak in the patient. This uncertainty can e.g. stem from uncertainty in the Hounsfield units of the CT image that is typically used for planning and the conversion of these Hounsfield units to stopping power.

Robustness Analysis (RA) is a technique for simulating the effect of range uncertainty and setup errors on the dose distribution of the IMPT plan. In RA the dose distributions for various error scenarios are calculated. Setup error is simulated by using anatomical data of the patient and three independent parameters that mimic the whole body movement of the patient (corresponding to X, Y and Z shifts). Range error can be entered as a percentage of uncertainty.

Robust Optimization (RO) is a technique for optimizing IMPT plans. This technique takes into account setup and range errors in order to calculate an IMPT plan with a reduced sensitivity to error. In RO an overall plan objective value, which includes a clinical objective component and a patient setup error component, is minimized.

WO2017/109632 describes a robust optimization technique that also includes possible errors due to organ deformation and movement within the patient. In this technique, the RO objective value that is minimized, includes additional components for organ movement and organ deformation.

In the current practice of IMPT planning, first a nominal plan is created that meets the clinical goals. This plan is then analyzed by RA. It is common for IMPT that the nominal plan will show localized areas with defects such as so-called hotspots or coldspots in the various simulated error scenarios. Robust optimization will improve the sensitivity to errors, and therefore resolve or reduce these deficiencies, but the overall quality of the plan is sacrificed in order to gain robustness. In cases where the nominal plan is acceptable and shows only small and/or few defects in the RA error scenarios, RO may lead to significant deterioration of the overall dose distributions. In these cases, it often happens that a physician will prefer to deliver the nominal plan without accounting for errors instead of delivering the RO plan. This means the treatment will then go ahead with the nominal plan and associated risks.

### SUMMARY OF THE INVENTION

The current invention seeks to provide the physician with an alternative or additional way to optimize a preferred initial therapy play. The current invention further seeks to address the need to improve the robustness of an IMPT plan for localized dose deficiencies whilst avoiding, or at least reducing, degradation of the overall plan quality.

It is a further insight of the invention that patient movement does not just occur during patient setup, but may also occur during treatment delivery. IMPT treatment can take significant time to deliver, e.g. half an hour. Within that time frame the patient may shift slightly, but more importantly TRs and OARs may move and change. The uncertainty in position that is due to these shifts and changes is referred to as local residual error (LRE). The current invention additionally or alternatively seeks to improve robustness of an IMPT plan for localized areas that have a high risk of LRE induced dose errors.

Further advantages from the described invention will also be apparent to the skilled person. Thereto a method and system for optimizing an IMPT plan are provided. Also, a computer program product comprising instructions which when executed control a processor to perform the method for optimizing an IMPT plan is provided. Further, an arrangement for planning IMPT is provided.

The method for optimizing an IMPT plan, comprising the steps of: receiving an initial IMPT plan for a subject to be treated; receiving anatomical image data of the subject to be treated; and identifying critical proton spots in the initial plan using the anatomical image data. Critical proton spots are the proton spots that have a high risk of delivering a deviant dose, e.g. too high or too low in view of the clinical goals, in case an error, such as a setup, range or LRE error, occurs. The method also comprises the step of generating at least one local plan deficiency area (LPDA) associated with the identified critical proton spots. A local plan deficiency area corresponds to the area in the dose map where the clinical goals, or similar clinically defined dose criteria, are not met in case an error occurs. The method further comprises the steps of generating a local treatment plan for the LPDA by applying robust optimization to the LPDA; and generating an optimized treatment plan for delivery to the subject to be treated by combining the local treatment plan with the initial IMPT plan.

In an examplary embodiment of the method, the critical proton spots are identified by applying RA to the initial plan. In this example, it can also be possible to identify the critical spots automatically based on automated RA and the received clinical goals.

According to another example of the method, critical proton spots are identified by calculating which proton spots have a local residual error risk value that is higher than a predetermined risk level.

In accordance with the method as desribed above and any of its examplary embodiments, the LPDA may, for example, be generated by defining an area around each critical proton spot representative of a location uncertainty of the critical proton spot and merging the areas around adjacent critical proton spots. Alternatively, the LPDA may be generated from graphical user input. According to another alternative, and for the example where the criticaly proton spots are identified by applying RA, the LPDA may be generated by identifying the locations of the critical proton spots in the initial plan and the locations of the shifted critical spots in the error scenario where these spots do not meet the clinical goals and encompassing these in a single area.

In accordance with the method as desribed above and any of its examplary embodiments, multiple LPDAs may be generated and a local treatment plan is generated for each LPDA.

The system for optimizing an IMPT plan comprises an input configured to receive an initial IMPT plan for a subject to be treated and an input configured to receive anatomical image of the subject to be treated. The system also comprises an identification module configured to identify critical proton spots in the initial plan using the anatomical image data, and an area calculation module configured to generate at least one local plan deficiency area associated with the critical proton spots identified by the identification module. The system further comprises a local treatment plan generator configured to apply RO to the LPDA, thereby generating a local robust optimized treatment plan for the local plan deficiency area; and an optimized treatment plan generator configured to combine the local treatment plan with the initial IMPT plan to form an optimized intensity modulated proton therapy plan for delivery to the subject to be treated.

In an exemplary embodiment of the system, the identification module comprises a robustness analysis user interface. Additionally or alternatively, the area calculation module may comprise a user interface.

In a particularly advantageous embodiment, one or both of the robustness analysis user interface and the area calculation module user interface are part of a graphical user interface. In such an embodiment, the graphical user interface can, for example, comprise a display for providing a visual representation of the anatomical image data with at least one of the dose distribution and the proton spot locations.

The computer program product comprises instructions which, when executed, control a processor to perform the method for optimizing an IMPT plan as described above.

The arrangement for planning IMPT comprising an imaging device configured to provide an image of a subject to be treated, a contouring tool configured to provide anatomical image data based on the image provided by the imaging device and an input for receiving clinical goals for the intensity modulated proton therapy. The arrangement further comprises a system configured to generate an initial intensity modulated proton therapy plan based on the anatomical data and the clinical goals; and the system for optimizing the IMPT plan as described above.

An advantage of the current invention is that the physician has more options available for optimizing an IMPT plan. With the current invention he, or she, can also use local robust optimization of a smaller area of the plan.

A further advantage of the current invention lies in that localized areas of the IMPT plan with a high risk of resulting in an inferior delivered dose due to errors are made more robust without adversely affecting the dose distribution of the areas plan of the plan that do not have such a risk. Because the invention identifies the critical proton spots and translates these into local plan deficiency areas, the process of robust optimization can be performed on such a high risk localized area only, leaving the remaining parts of the plan unaffected.

Another advantage lies in that the IMPT plan can be made more robust with respect to possible patient movement and/or organ movement and deformation that occurs during treatment delivery. By identifying the critical spots that have a high risk for inferior dose delivery due to LRE, the areas corresponding to these spots can be subjected robust optimization.

### BRIEF DESCRIPTION OF THE FIGURES

In the following drawings:
Fig. 1 schematically and exemplarily illustrates the particular shape of a Bragg-peak for a proton beam.
Fig. 2 schematically illustrates an example of an arrangement for planning IMPT that comprises a system for optimizing an IMPT plan.
Fig. 3 schematically illustrates an example of an initial plan generator.
Fig. 4 schematically and exemplarily illustrates a method for optimizing an IMPT plan for localized deficiencies.
Fig. 5a and 5b schematically illustrate and exemplarily illustrate using RA to identify critical spots and generating an LPDA.
Fig. 6 and 6b schematically illustrate an example where critical proton spots are identified by calculating which proton spots have a high riske for LRE.
Fig. 7 schematically illustrates an example where wherein the LPDA is generated by defining an area of uncertainty around each critical proton spot and merging these areas into a smooth shape.

### DETAILED DESCRIPTION OF THE INVENTION

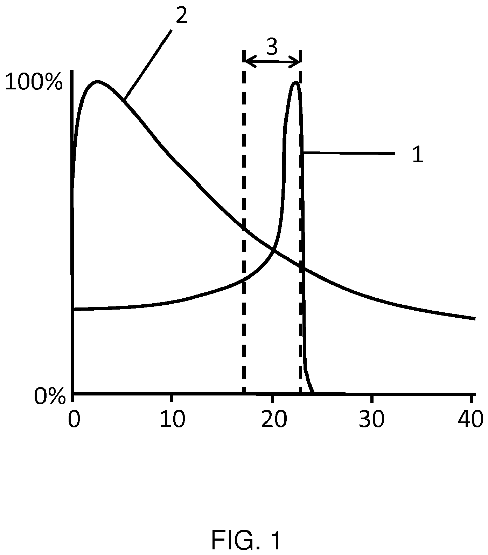

Fig.1 illustrates the shape of a Bragg-peak for a proton beam 1. Beam intensity is shown as a function of penetration depth. For comparison, the intensity profile of a conventional X-ray beam 2 used for radiation therapy is also shown. In this figure, the vertical axis shows percentage of intensity and the horizontal axis shows penetration depth in cm. The target structure 3 is located between the dashed lines around 20 cm beneath the patient's skin. As can be seen in the figure, the proton beam 1 delivers a high intensity within the target structure 3 and a lower intensity at and directly beneath the skin. In particular when compared to the conventional X-ray beam 2. Fig. 1 also shows the sharp fall-off of the intensity of the proton beam at the right-hand side of the target structure 3. This shape allows for high precision of delivery compare to the conventional X-ray beam 2, but it also has particular sensitive to errors such as range and setup uncertainties. In case the target structure 3 is displaced by several millimeters, the high intensity peak of the proton beam 1 could be located just outside the target structure.

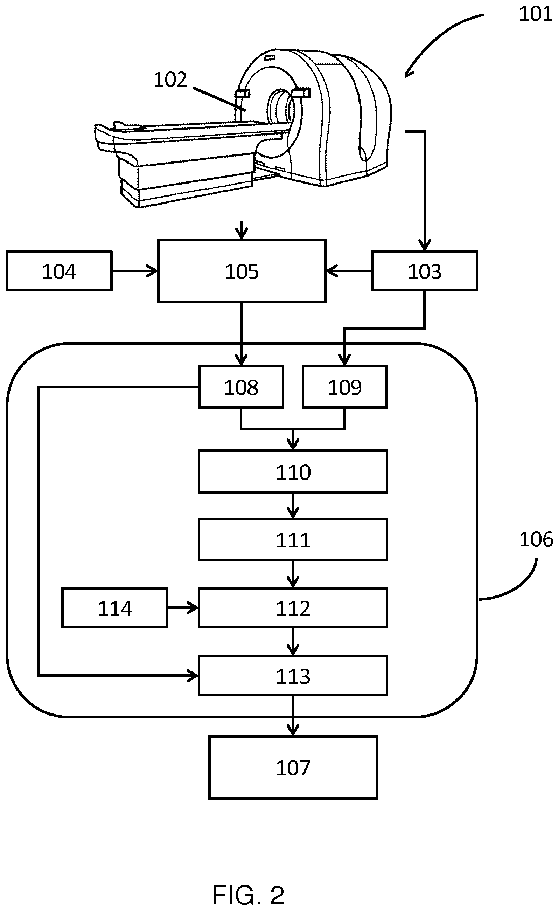

Fig.2 schematically illustrates an example of an arrangement 101 for planning IMPT that comprises a system for optimizing an IMPT plan. The arrangement 101 has an imaging device 102 that is configured to provide an image of a subject to be treated, a contouring tool 103 that is configured to provide anatomical image data based on the provided image, an input for receiving clinical goals 104 for the IMPT plan, an initial plan generator 105 and a system for optimizing the IMPT plan 106. The output of this arrangement is an optimized IMPT plan 107.

The imaging device 102 of the arrangement can be a structural imaging device, such as a CT, MR or ultrasound scanner, a functional imaging device, such as a PET or SPECT scanner or any combination thereof. It has particular advantages to use a combined PET-CT scanner, because a PET-CT image will allow the physician to identify OARs from the structural CT image as well as cancerous TSs from the PET functional image.

The image of the subject to be treated that is provided by the imaging device 102 is used as input for a contouring tool 103. The contouring tool delineates the OARs and TSs that are identified in the image. These delineations form the contours that the anatomical image data for the subject to be treated. This delineation is commonly referred to as contouring. The contouring tool 103 may identify the contours automatically, or it may have an interface where the physician or a trained clinician inputs the contours manually. The contouring tool 103 may also use a combination of manual and automated contouring.

In order to generate the plan for the IMPT, clinical goals for the treatment are needed. The arrangement provides an input 104 for these goals. The clinical goals can be determined by the physician based on the image provided by the imaging device 102, and may be based on clinical protocol. The clinical goals can be entered manually through a user interface, or, for example, uploaded automatically from a database using anatomical image data. Clinical goals will typically include maximum dose limits for the identified OARs and a minimum or average dose to be received by a TS.

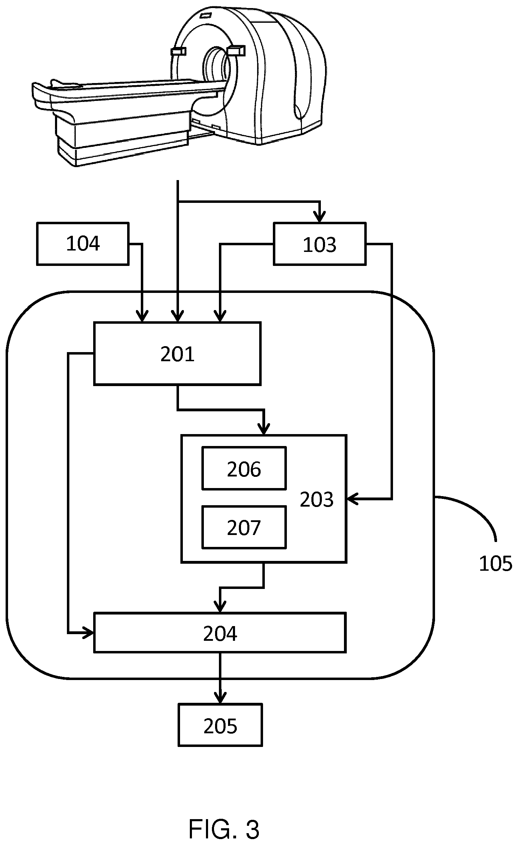

The initial plan generator 105 is a system that is configured to generate an initial IMPT plan. For generating the initial plan, a commercially available product such as Auto Plan may be used. Generating the initial IMPT plan is usually an interactive process performed by a physician and will be described in more detail with reference to Fig.3.

The initial IMPT plan provided by the initial plan generator 105 and anatomical image data provided by the contouring tool 103 are then used as input for the system for optimizing the IMPT plan 106. For this, the system 106 has an input 108 configured to receive the initial IMPT plan for the subject to be treated and an input 109 configured to receive the anatomical image data of the subject to be treated. These inputs may also be combined into a single input. In the example of figure 2, an embodiment of the arrangement for planning IMPT is shown where the initial plan generator 105 and contouring tool 103 are separate units to the system 106 for optimizing the IMPT plan. In such a setup, the inputs 108 and 109 could be external data ports. In alternative embodiments, the plan generator 106, the contouring tool 103 and the system 106 could also be part of only two or even a single unit. In the example of a single unit, inputs 108 and 109 would be part of an automated internal data transfer.

The system further has an identification module 110 configured to identify the critical proton spots in the initial plan using the anatomical image data and an area calculation module 111 configured to generate at least one LPDA associated with the critical proton spots that have been identified by the identification module 110. Next, a local treatment plan generator 112 applies RO to the LPDA and thereby generates a local robust optimized treatment plan for the LPDA. The robust optimized local treatment plan can be generated in a manner that is similar to the generation of an overall robust optimized plan, except calculations are performed on the LPDA only. For generating the local robust optimized treatment plan, the system 106 may comprise an input for local clinical goals 114. This example is illustrated in Fig.2. Alternatively, the input for the local clinical goals 114 may be the same as the input for the clinical goals for the initial plan 104. In a further alternative, no additional or alternative local clinical goals are provided for the LPDA. In such an embodiment, the clinical goals of the initial plan from input 104 can be used. In such a case, these goals are received by input 108 along with the initial plan and input 108 is then also configured to receive the clinical goals.

The system 106 also comprises an optimized treatment plan generator 113 that is configured to combine the local treatment plan with the initial IMPT plan. Hereby the optimized IMPT plan 107 for delivery to the subject to be treated is formed.

Fig.3 illustrates an example of an initial plan generator 105 in more detail. The system 105 that is configured to generate the initial IMPT plan uses the anatomical image data from the contouring tool 103 and the clinical goals from the input 104. In the embodiment shown in Fig.3, the image of the subject to be treated that is provided by the imaging device 102 is also used by the initial plan generator 105. Although the image is not essential for generating an IMPT plan, it can provide helpful additional visual guidance to the physician who is determining the plan.

The embodiment of system for generating the initial IMPT plan illustrated in Fig.3 comprises a nominal plan generator 201, a global RO unit 203, and a plan selector 204. The output of the system is the selected initial plan 205.

The nominal plan generator 201 will generate a nominal IMPT plan in the manner that is commonly known in the art. This is an interactive process in which a dose distribution is calculated using numerical optimization based on the anatomical image data and the clinical goals provided by the physician. The physician assesses the dose distribution and adjusts the clinical goals until the dose distribution is acceptable for delivery to the subject to be treated. From this dose distribution the nominal plan generator 201 then calculates the parameters for delivery to form the nominal IMPT plan.

In a simple embodiment of a system for generating an initial IMPT plan 105, this system can consist of a nominal plan generator 201. In this simple case, the nominal plan is the initial plan. However, in a more extensive embodiment, it can be advantageous to also include a global RO unit 203 and a plan selector 204. The global RO unit 203 allows the physician to analyze the overall robustness of the nominal IMPT plan using an RA interface 206. With this interface, the physician can simulate the various error scenarios including range and setup error. The global RO unit can then use an RO calculator 207 on the nominal plan to calculate an RO plan. This may be preferred in cases where the RA shows many and/or extensive areas of dose deficiencies. Or the physician may simply wish to assess how much of the overall plan quality is sacrificed to reduce error sensitivity in the RO of the overall plan.

In order to make a choice as to which IMPT plan to use as the initial plan 205, the initial plan generator 105 comprises a plan selector 204. The plan selector presents the options of choosing between the nominal IMPT plan generated by the nominal plan generator 201 and the RO plan generated by the global RO unit 203. The plan that is chosen is then the initial plan 205 that is the output. It is particularly useful for the plan selector 204 to be operable independent of the global RO unit 203, meaning that a choice can be made irrespective of whether a global RO plan was calculated or not. The advantage of this is that the physician can bypass the RO of the full plan and directly choose the nominal plan as initial plan 205 if that is his preference.

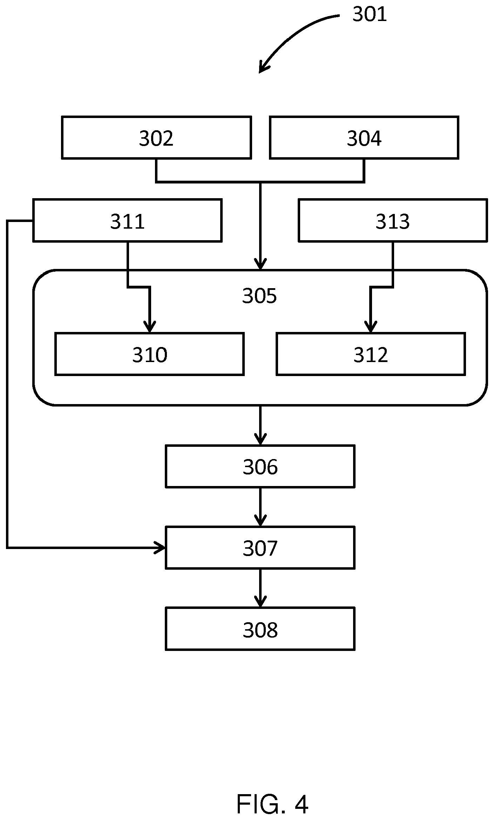

Fig.4 schematically illustrates a method for optimizing an IMPT plan 301. In this method the plan is optimized for localized deficiencies.

In the method for optimizing the IMPT plan, an initial IMPT plan is received as input at 302. Such an initial IMPT plan may be the nominal IMPT plan, but it can also be an IMPT plan that has already undergone global RO. The initial plan can, for example, be determined by using the initial plan generator illustrated in figure 3. Alternatively, the initial plan may also be predetermined and uploaded from a database such as a patient information database.

In the method illustrated in Fig.4, anatomical image data is also received as input 304. Such anatomical image data may, for example, be provided by an automated or an interactive contouring tool. In that case the anatomical image data comprises contours of at least one OAR and at least one TS. Also the anatomical image data can alternatively be predetermined and uploaded from a database such as a patient information database.

Next, critical proton spots are identified in the initial plan using the anatomical image data 305. Critical proton spots are the proton spots that have a high risk of delivering a deviant dose, e.g. too high or too low in view of clinical goals, in case an error occurs. Such an error can be a range or setup error, an error due to organ motion or deformation, or an LRE.

In the method for optimizing an IMPT plan, critical proton spots may be identified in several ways. Fig.4 illustrates two examples for identifying critical proton spots that can be used separately or in combination.

In one example, the critical proton spots are identified by applying RA to the initial plan 310. This can be done interactively by the physician or automatically. In the option where RA is applied interactively by the physician, preferably a user interface is provided, more preferably in the form of a graphical user interface. Such an interface will allow the user to input the error scenario he wishes to simulate and he or she can the see the effect of the error on the dose distribution. The physician can the identify the areas in the dose distribution where the delivered dose would be deviant because it is too high or too low. An area with a dose that is too high would show as a hot spot, an area where the dose is too low as a cold spot. In such an interactive system, the proton spots could also be displayed and the physician could mark them as critical by clicking on them. In an alternative option, the user could be provided with a drawing tool for drawing around the area of dose deviation and the critical proton spots are then defined as the proton beams that would be delivered in that area. In the option where RA is performed as an automated process, the clinical goals are received 311 as additional input. Critical spots are then identified based on this automated process and the received clinical goals 301. In this case a spot is identified as critical in case, in one or more of the error scenarios, the dose that is delivered by the proton beam does not meet the clinical goals.

In a second example, the critical proton spots are identified by calculating which proton spots have local residual error risk value that is higher than a predetermined risk level 312. In this case, the predetermined risk level may be a preset threshold value, but it can also be received as an additional input value 313.

With the identified critical proton spots, at least one LPDA is created 306. A local treatment plan is then generated for the LPDA by applying RO to the LPDA 307. This treatment plan is local in nature, because it relates specifically to the LPDA and not to the IMPT plan as a whole. In generating this treatment plan, RO is applied specifically to the LPDA and not to the IMPT plan as a whole. In an advantageous embodiment the clinical goals that have been received as input are also used when RO is applied to the LPDA. This allows for a fully automated IMPT plan optimization process. Alternatively, additional or alternative clinical goals may be received at 307 for the process of applying RO to the LPDA.

The distribution of critical spots may also be such that it is preferred to generate multiple LPDAs. For example, a plan may show critical protons spots causing a hot spot and a cold spot in the delivered dose two separate locations in an RA error scenario, or multiple small hot or cold spots in the delivered dose different locations or for different error scenarios. In this case it could be preferred to generate an LPDA for each of these small areas of dose deviations. Alternatively, for example, there may also be two or more areas with critical proton spots with a high risk for LRE. In this case it could be preferred to generate an PLDA for each area of critical proton spots with a high risk for LRE. An IMPT plan may also have a combination of critical proton spots for areas of dose deviations in RA error scenarios with critical proton spots with a high risk for LRE. In such a case, it is preferred that the spatial locations and proximities of the various critical spots will determine whether a single LPDA should be generated or whether two or more LPDAs should be generated. If the critical proton spots are clustered in one smaller region, a single LPDA will suffice for all critical proton spots, regardless of their origin. However, if there are two or more spatially separate clusters, creating multiple LPDAs is preferred. In case multiple LPDAs are generated, a local treatment plan is generated 307 for each of the LPDAs by applying RO to each area. The result is that each LPDA has its own RP optimized local treatment plan.

When the local treatment plan or plans have been generated, it is, or they are, combined with the initial IMPT plan to generate the optimized IMPT treatment plan 308 for delivery to the subject to be treated. A possible way of combining a local treatment plan with the initial treatment plan is replacing the delivery parameter for the critical proton spots in the initial IMPT plan with the newly determined delivery parameters of the local treatment plan whilst leaving the delivery parameters for the other, remaining proton spots unchanged.

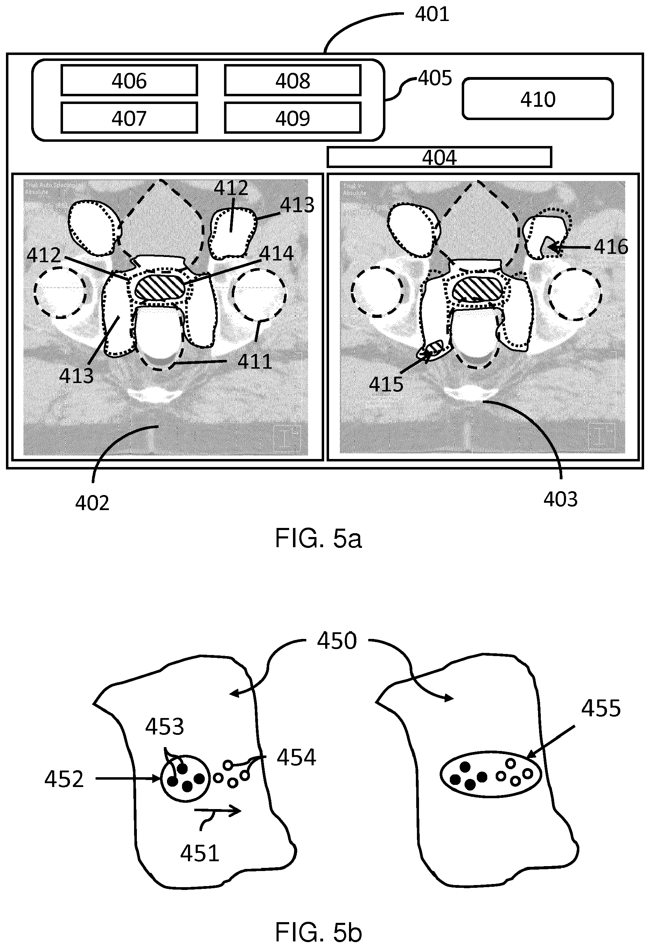

Figs. 5a and 5b schematically illustrate and exemplarily illustrate using RA to identify critical spots and generating an LPDA.

Fig.5a shows an embodiment of a system for RA. The system of this embodiment has a graphical user interface 401. The interface shows the dose distribution according to the initial IMPT plan in a display on the left and the dose distribution according to the error scenario 403 in the display right. The error scenario has an information field 404 providing the parameters used in the simulation that is visualized. The interface further has controls 405 where the physician can input the error scenario he or she wishes to investigate. In this embodiment the controls have an input 406 for the range error as a percentage, an input 407 for left-right x-displacement in mm, an input for up-down y-displacement in mm and input for z-displacement that is perpendicular to the figure in mm. The inputs can have several forms such as text boxes, numerical input boxes, sliders or a combination thereof. In this embodiment the interface also has a button 410 that will start a new dose distribution calculation for the current values of the inputs 406, 407, 408 and 409.

In the exemplary embodiment of Fig.5a, the initial plan and error scenario displays 402 and 403 show anatomical image data and dose distribution on a background of an image received from an imaging device. This image has the advantage of providing additional guidance for the physician, but is not essential for the analysis. The anatomical image data is shown in the displays as contours of the OARs 411 in dashed lines and contours of the TSs 412 in dotted lines. In Fig.5a, the dose distribution is visualized by isodose lines with are fill. A medium level dose is shown by the white area 413 with a demarcation line and a high level dose is shown by a hatched area 414 with a demarcation line. An alternative, and more commonly used way of visualizing the dose is by using a color scale where red represents a high intensity, yellow a medium intensity and blue a low intensity.

In the error scenario depicted in display 403 of the RA system of Fig.5a a shift of the patient of several mmm to the right has been simulated. It can be clearly seen that without modification, delivery of the initial IMPT plan would result in a hot spot 415, and a cold spot 416. The proton spots causing the unintended area of high dosage 415 are now identified as critical proton spots. The proton spots causing the unintended area of low dosage 416 are also identified as critical proton spots. These critical proton spots can be identified automatically based on the clinical goals, which are not met for areas 415 and 416. The critical proton spots can also be identified by the physician using a drawing tool as in put in the display 403. Using a drawing tool has the additional advantage of providing the physician with additional control over the identification. The identified spots can be stored as part of a list until all envisaged error scenarios have been simulated an all critical spots for each identified. At least one LPDA can now be generated. In view of the clustering and location of the critical proton spots that can been seen in display 403, in this example it will be preferred to generate two LPDAs: one for the hotspot 415 and one for the coldspot 416.

Fig.5b shows a particular embodiment for generating an LPDA for the situation where RA has been used to identify the critical proton spots. In this example the subject to be treated has a TS 450. The left side of the figure shows the TS in the error scenario where the subject has a setup error and has shifted to the right as indicated by arrow 451. In case the initial plan would be delivered without further modification, this shift would cause a hot spot 452 in the dose distribution. The dose delivered in this hotspot is deviant because it is too high. The proton spots that result in the hot spot, solid circles 453, are thereby identified as critical proton spots. The location where these spots would have been delivered with respect to the TS 450 without the subject's shift is indicated by open circles 452. The LPDA 455 is now generated by identifying the locations of the critical spots in the initial plan 454 and identifying the locations of the shifted critical spots 453 and encompassing these in a single area. In this example, the spots are encompassed in a smooth elliptical shape, but alternative shapes can also be used. Preferably the shape is a smooth shape such as a circle, ellipse, or rounded rectangle. The shape may be automatically selected and fitted to the area or this can be done interactively by the user. In an advantageous alternative embodiment, when the LPDA is generated by an area calculation module, this module has a user interface for fitting the shape to the area. In such an embodiment it could also be possible to provide the physician with a drawing tool for inputting the LPDA. It would be even more advantageous for the LPDA user interface to be combined with the RA interface. In such an embodiment, the physician can investigate the coverage of the LPDA in several different error scenarios.

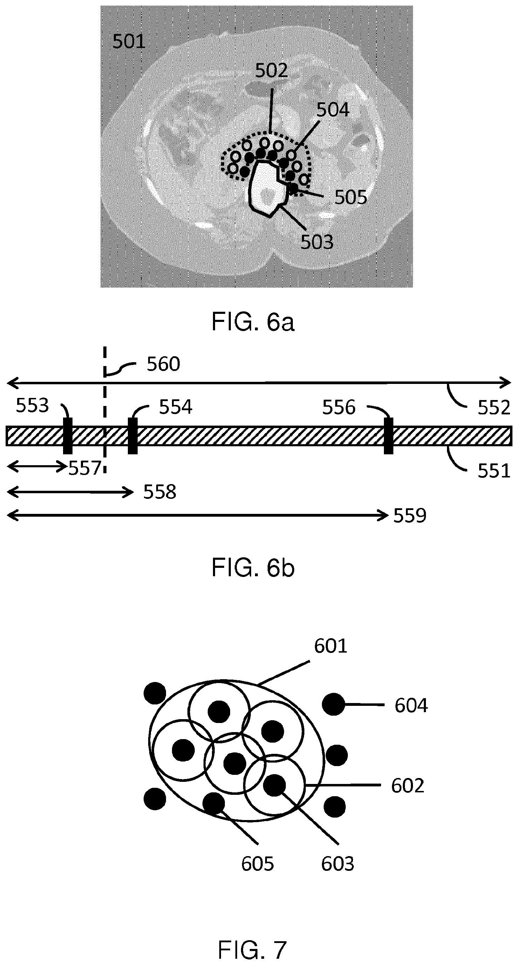

Figs. 6a and 6b schematically illustrate an example where critical proton spots are identified by calculating which proton spots have a high risk for LRE.

Within the time span that IMPT takes to deliver, or, when the treatment is split into multiple fractions, the time span that a fraction of the IMPT takes to deliver, the subject that is treated may shift slightly, and TRs and OARs may move and change. LRE is the uncertainty in position that is due to these shifts and changes and the magnitude of the LRE during treatment delivery increases with time. This also means that the risk of delivering a deviant dose increases with time. Proton spots that are delivered earlier during the treatment will be affected less by LRE than proton spots.

The current invention additionally or alternatively seeks to improve robustness of an IMPT plan for localized areas that have a high risk of LRE induced dose errors. For this purpose, the critical proton spots that have a high risk for LRE are identified. This process comprises two steps.

First, the potential critical proton spots are identified based on their location. For many locations in the patient's body, delivery of the proton spot in accordance with the initial plan will not result in the delivery of a deviant dose in case an LRE occurs. The proton spots that are delivered to these locations are low-risk with a low LRE risk value and are non-critical proton spots. Proton spots that can result in the delivery of a deviant dose in case an LRE occurs are typically located in proximity to an OAR and a TS. Fig.6a shows an example of the identification of high and low LRE risk proton spots based on location. A background CT is mage 501 is shown with an overlay of the dashed contour of a TS 502 and the solid contour an AOR 503, which in this example is the spine. The open circles 504 are proton spots that are sufficiently removed from the spine to be low-risk LRE spots and these are classified as non-critical. Closed circles 505 are in proximity of both the spine 503 and the TS 502 and are in a high-risk location. These spots are identified as potential critical proton spots.

After these potential critical proton spots have been identified based on location, the time of delivery is calculated. The probability that LRE occurs, and thereby the LRE risk value, increases with the time taken to deliver the proton spot. Recent research suggests that the risk increases linearly with time and the risk can therefore be calculated by multiplying the delivery time with a predetermined proportionality risk constant. In order to determine to moment of delivery of each proton spot, the complete delivery of the treatment is simulated.

The combination of location of the proton spot with the moment of delivery during the treatment determines the LRE risk value of that proton spot. A visual representation of a simulation delivery is illustrated in Fig.6b. When the value is higher than a predetermined risk level, the proton spot is identified as a critical proton spot. All delivered proton spots in the fraction of the IMPT treatment that is delivered are represented by dashed bar 551 with a total time of delivery 552. Proton spots 553, 554 and 556 are in locations with a high risk for LRE induced dose errors. It is now calculated that these proton spots are delivered by proton beams around time intervals 557, 558 and 559. Because the risk for LRE induced error increases linearly with time, a predetermined LRE threshold corresponds to a threshold time interval. In this example, it has been determined that LRE exceeds the predetermined threshold at time interval 560. This means that the LRE risk value of proton spot 553 is below the predetermined risk level and it is therefore not a critical proton spot. The LRE risk values of proton spots 554 and 556 are above the predetermined risk level and these two are therefore identified as critical proton spots.

In the example of Fig.6b, all potential spots identified by location are treated with equal weight and their LRE risk value is determined by the delivery time. This approach may additionally be refined by adding a weight factor that depends on proximity to an OAR. Such an approach may be beneficial in cases where proton spots are in a high risk location and close to an OAR as well as close to the threshold for delivery time. Close proximity to an OAR will in such an example and increase the risk factor to allow inclusion of the proton spot in the LPDA.

Fig.7 illustrates an embodiment where wherein the LPDA is generated by defining an area of uncertainty around each critical proton spot and merging these areas. Preferably this is done automatically using a maximum uncertainty value for the delivery location of the proton spot. As show in Fig.7, the area of uncertainty may be defined by a circle with the critical proton spot 603 a the center and a radius with the size of the uncurtainty value. The LPDA can be created by merging the circles into a single shape by cropping the compund shape formed by all circles together. In that case, the LPDA will have the minimun required area and an irregular outer contour. An alternative option is shown in figure 7. Here the LPDA is created by merging the circles into a smooth elliptical shape that is fitted tightly around the uncertainty areas. All critical proton spots 603 are encompassed in the LPDA 601. In this approach a small number of non-crtical proton spots 605 may also fall within the LPA, but most non-critical proton spots 604 will remain outside it.

Any of the method steps disclosed herein, may be recorded in the form of a computer program comprising instructions which when executed on a processor cause the processor to carry out such method steps. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray™ and DVD. Examples of a propagation medium are the Internet or other wired or wireless telecommunication systems.

Variations to the disclosed embodiments can be understood and effected by those skilled in the art in practicing the claimed invention, from a study of the drawings, the disclosure, and the appended claims. It is noted that the various embodiments may be combined to achieve further advantageous effects.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A method for optimizing an intensity modulated proton therapy plan (301), the method comprising:
- Receiving an initial intensity modulated proton therapy plan (302) for a subject to be treated;
- Receiving anatomical image data of the subject to be treated (304);
- Identifying critical proton spots in the initial plan using the anatomical image data (305);
- Generating at least one local plan deficiency area (306) associated with the identified critical proton spots;
- Generating a local treatment plan (307) for the local plan deficiency area by applying robust optimization to the local plan deficiency area; and
- Generating an optimized treatment plan for delivery to the subject to be treated by combining the local treatment plan with the initial intensity modulated proton therapy plan (308).

2. The method according to claim 1, wherein the critical proton spots are identified by applying robustness analysis to the initial plan (310).

3. The method according to claim 2, wherein the critical spots are identified automatically based on automated robustness analysis and received clinical goals.

4. The method of claim 1, wherein critical proton spots are identified by calculating which proton spots have a local residual error risk value that is higher than a predetermined risk level (312).

5. The method according to any of claims 1-4, wherein the local plan deficiency area (601) is generated by defining an area (602) around each critical proton spot (603) representative of a location uncertainty of the critical proton spot and merging the areas around adjacent critical proton spots.

6. The method according to any of claims 1-4, wherein the local plan deficiency area is generated from graphical user input.

7. The method according to claim 2 or 3, wherein the local plan deficiency area (455) is generated by identifying the locations of the critical proton spots in the initial plan(454) and the locations of the shifted critical spots in the error scenario (453) where these spots do not meet the clinical goals and encompassing these in a single area.

8. The method according any of claims 1-7, wherein multiple local plan deficiency areas are generated and a local treatment plan is generated for each local plan deficiency area.

9. A system for optimizing an intensity modulated proton therapy plan (106), the system comprising:
- An input configured to receive an initial intensity modulated proton therapy plan for a subject to be treated (108);
- An input configured to receive anatomical image of the subject to be treated (109);
- An identification module configured to identify critical proton spots in the initial plan using the anatomical image data (110);
- An area calculation module configured to generate at least one local plan deficiency area associated with the critical proton spots identified by the identification module (111);
- A local treatment plan generator configured to apply robust optimization to the local plan deficiency area, thereby generating a local robust optimized treatment plan for the local plan deficiency area (112); and
- An optimized treatment plan generator configured to combine the local treatment plan with the initial intensity modulated proton therapy plan to form an optimized intensity modulated proton therapy plan for delivery to the subject to be treated (113).

10. The system of claim 9, wherein the identification module comprises a robustness analysis user interface (401).

11. The system of claim 9 or 10, wherein the area calculation module comprises a user interface.

12. The system of claims 10 or 11, wherein one or both of the robustness analysis user interface and the area calculation module user interface are part of a graphical user interface.

13. The system of claim 12, where the graphical user interface comprises a display for providing a visual representation of the anatomical image data with at least one of the dose distribution and the proton spot locations.

14. A computer program product comprising instructions which when executed control a processor to perform the method according to of any one of the claims 1-8.

15. An arrangement for planning intensity modulated proton therapy (101) comprising:
- an imaging device (102) configured to provide an image of a subject to be treated;
- a contouring tool (103) configured to provide anatomical image data based on the image provided by the imaging device;
- an input for receiving clinical goals (104) for the intensity modulated proton therapy;
- a system configured to generate an initial intensity modulated proton therapy plan (105) based on the anatomical data and the clinical goals; and
- the system (106) according to any of claims 9-13.
